# EUROPEAN PATENT APPLICATION

(11) **EP 4 424 813 A1**
(43) Date of publication of application: **04.09.2024**
(21) Application number: 22894685.1
(22) Date of filing: 09.11.2022
(51) Int. Cl.: C12M 1/34, C12M 1/00, B01L 3/00

(54) **INTEGRATED MICRODROPLET CHIP**

(30) Priority: 20.11.2021 CN 202111381092
(71) Applicant: Targetingone Technology (Beijing) Corporation, Beijing 102200 (CN)
(72) Inventor: SU, Shisheng, Beijing 102200 (CN); WANG, Bo, Beijing 102200 (CN); XIA, Lei, Beijing 102200 (CN); LIU, Jinwei, Beijing 102200 (CN); YANG, Wenjun, Beijing 102200 (CN); WANG, Yongdou, Beijing 102200 (CN)
(74) Representative: LLR
(86) International application number: PCT/CN2022/130910
(87) International publication number: WO 2023/088148

(57) **Abstract**

The invention discloses an integrated digital PCR instrument and a control method therefor. The integrated digital PCR instrument comprises a chip loading module (200); a droplet generation module (300), which can generate droplets (4) from a sample in a sample adding chamber (12) during the droplet generation process, and then the droplets being transferred and stored in a reaction chamber (11); a temperature cycle module (400), which enables the droplets (4) in the reaction chamber (11) to achieve amplification; a fluorescence detection module (500), which can transfer the droplets (4) in the reaction chamber (11) to a fluorescence detection area (33) during a process of detecting the droplets (4), then transfers the droplets to the sample adding chamber (12), and completes fluorescence detection of the droplets (4) during said process; a chip disposal module (600); and a scheduling mechanism (700), which is used for transferring an integrated droplet chip (100) among the chip loading module (200), the droplet generation module (300), the temperature cycle module (400), the fluorescence detection module (500) and the chip disposal module (600) under the control of a main control module (800), and can turn up and down by 180° the integrated droplet chip (100) in the temperature cycle module (400) and then transfers the integrated droplet chip into the fluorescence detection module (500). The degrees of automation and integration of the PCR instrument are high, which can improve detection and analysis operation efficiency and reduce labor costs.

## Description

### TECHNICAL FIELD

The present invention relates to the technical field of digital PCR analysers, and particularly relates to an integrated droplet chip.

### BACKGROUND

Droplet-based microfluidics is a technology platform developed in recent years on microfluidic chips to manipulate small volumes of liquid, and its principle is: two immiscible liquids, illustratively, one of which is an oil phase and the other is an aqueous phase, are allowed to enter a microchannel at the same time. Under the action of the microchannel, the aqueous phase is distributed in the oil phase in the form of small volume units, forming a series of discrete droplets. Each droplet acts as a microreactor, completing a set of chemical or biological reactions.

Digital PCR technology is regarded as the third generation PCR technology, with the advantages of absolute quantification and single molecule detection sensitivity, which has an important application prospect in the field of molecular diagnosis. A mainstream technology route of Digital PCR technology is to use droplet microfluidic chips, the reaction system will be divided into tens of thousands or even millions of droplets of uniform size, and complete the generation, amplification and fluorescence detection, The precise copy number of the target molecule in the sample is calculated using mathematical models based on the fluorescence detection results.

In droplet digital PCR technology, the structure in the droplet generation chip is often used to complete the droplet generation, then the droplets are transferred to the reaction tube for amplification, and finally the structure in the droplet detection chip is used to form a droplet queue with certain intervals, and each droplet passes through the fluorescence detection area in turn to excite and detect fluorescence signals within the droplets. This method of using droplet microfluidics to achieve digital PCR has the advantages of uniform droplet size, the number of droplets is not easy to be limited, and the signal-to-noise ratio of fluorescence detection is high, but it also has the shortcomings of complex chip structure, the generation and detection are done in different chips, the integration is low, and it is difficult to be automated.

### SUMMARY OF THE INVENTION

Therefore, the technical problem to be solved by the present invention is to provide an all-in-one droplet chip to overcome the shortcomings of the prior art in which droplet generation and detection are done in different droplet chips, resulting in low integration and automation.

In order to solve the above problems, the present invention provides an integrated droplet chip, it comprises a chip body, the chip body has a reaction chamber and a sample adding chamber, and said chip body being constructed with droplet generating structure, oil-liquid interface, gas-liquid interface and fluorescence detection area, the gas-liquid interface communicates with the reaction chamber, and the sample adding chamber communicates with the droplet generating structure, and the oil-liquid interface communicates with the droplet generating structure;
when the droplets are generated, a first pressure difference is formed between the sample adding chamber and the gas-liquid interface, and a second pressure difference is formed between the oil-liquid interface and the gas-liquid interface, the first pressure difference and the second pressure difference respectively drive the sample in the sample adding chamber and the generated oil of the oil-liquid interface to enter the droplet generating structure, the generated droplets enter and are stored in the reaction chamber;
during the droplet detection, the external pressure drives the detection pushing oil to enter the reaction chamber from the gas-liquid interface, so that the droplets in the reaction chamber flow out of the reaction chamber and enter into the droplet generating structure, and the external pressure drives the detection separation oil to enter the droplet generating structure from the oil-liquid interface, and the detection separation oil separates the droplets flowing out of the reaction chamber into the droplet generating structure to form a queue, which enters the fluorescence detection area.

In some embodiments, the droplet generating structure comprises an oil-liquid pipeline and a communication pipeline, the oil-liquid pipeline intersects with the communication pipeline in a cross manner, the communication pipeline comprises a first pipeline located on a first side of the cross point and communicated with the reaction chamber, and a second pipeline located on a second side of the cross point and communicated with the sample adding chamber, and the oil-liquid interface is communicated with the oil-liquid pipeline.

In some embodiments, the reaction chamber and the sample adding chamber are located on the first side surface with reference to the first side surface of the chip body in a horizontal direction, and the connecting interface between the reaction chamber and the first side of the chip body extending upwards and forming a flared mouth with a small upper part and a large lower part.

In some embodiments, a gas-liquid pipeline extending from bottom to top is further configured in the reaction chamber, a lower opening of the gas-liquid pipeline is in communication with the gas-liquid interface, and an upper opening of the gas-liquid pipeline is higher than an upper opening of the connecting interface.

In some embodiments, a droplet observation area is provided between the first pipeline and the connecting interface.

In some embodiments, the sample adding chamber comprises an opening chamber and a sealing cover hermetically connected to said opening chamber.

In some embodiments, the sample adding chamber is provided with a filter membrane or an exhaust hole.

In some embodiments, the fluorescence detection area is located on the second pipeline.

In some embodiments, the sample adding chamber is on a first side surface and the reaction chamber is on a second side surface, said first side surface and said second side surface being opposite sides of said chip body.

In some embodiments, light oil is preset in the reaction chamber before the droplets enter the reaction chamber.

The present invention provides an integrated droplet chip, wherein said chip body is integrated with a sample adding chamber, a reaction chamber, a droplet generating structure, and a fluorescence detection area, and wherein droplet generation, amplification, and detection are all integrated in a single chip through time-sharing multiplexing of said droplet generating structure, to achieve a fully integrated and fully closed digital PCR process.

### BRIEF DESCRIPTION OF THE DRAWINGS

Figure 1 shows a schematic diagram of a three-dimensional structure of an integrated droplet chip of an embodiment of the present invention;
FIG. 2 shows a structural schematic diagram of a droplet generating structure in the integrated droplet chip of an embodiment of the present invention;
Figure 3 shows a schematic diagram of the internal structure of the reaction chamber in the integrated droplet chip of the embodiment of the present invention;
Figure 4 shows a schematic diagram of the droplet generation process;
FIG. 5 shows a schematic diagram of the droplets stored in the reaction chamber after generation;
Figure 6 shows a schematic diagram of the state inside the reaction chamber after the integrated droplet chip is turned over by 180°;
Fig. 7 shows a schematic representation of the state in the state of Fig. 6 after an oil is passed into the reaction chamber;
FIG. 8 is a schematic illustration of the state of the droplet after it is forced out of the reaction chamber (the arrows in the figure show the droplet and the direction of flow of the oil liquid);
Figure 9 shows a schematic diagram of the three-dimensional structure of the integrated droplet chip of another embodiment of the present invention.

The reference numeral is expressed as:
1, chip body; 11, reaction chamber; 111, connecting interface; 112, gas-liquid pipeline; 12, sample adding chamber; 121, opening chamber; 122, sealing cover; 21, oil-liquid pipeline; 22, first pipeline; 23, second pipeline; 31, oil-liquid interface; 32, gas-liquid interface; 33, fluorescence detection area; 34, droplet observation area; 4, droplet; 5, detection pushing oil.

### DETAILED DESCRIPTION OF THE EMBODIMENTS

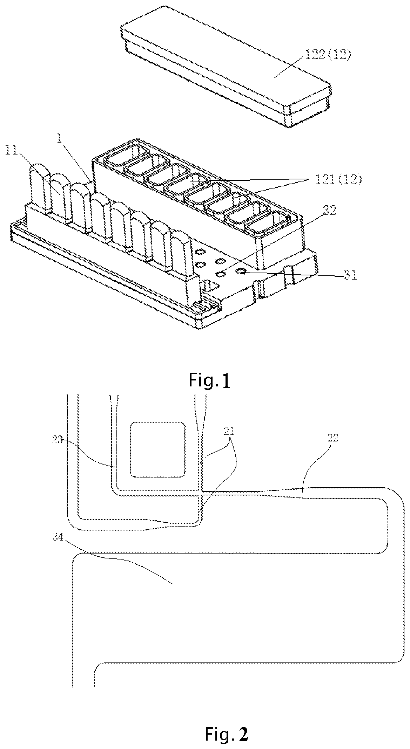

Referring to FIG. 1 to FIG. 9, according to an embodiment of the present invention, an integrated droplet chip is provided, comprising a chip body 1, wherein the chip body 1 has a reaction chamber 11 and a sample adding chamber 12, wherein a droplet generating structure, an oil-liquid interface 31, a gas-liquid interface 32 and a fluorescence detection area 33 are constructed in the chip body 1, wherein the gas-liquid interface 32 is in communication with the reaction chamber 11, the sample adding chamber 12 is in communication with the droplet generating structure, and the oil-liquid interface 31 is in communication with the droplet generating structure.

When droplets are generated, a first pressure difference is formed between the sample adding chamber 12 and the gas-liquid interface 32, and a second pressure difference is formed between the oil-liquid interface 31 and the gas-liquid interface 32. The first pressure difference and the second pressure difference respectively drive the sample in the sample adding chamber 12 and the generated oil of the oil-liquid interface 31 to enter the droplet generating structure, and the generated droplets 4 enter and are stored in the reaction chamber 11.

During droplet detection, external pressure drives the detection pushing oil 5 to enter the reaction chamber 11 from the gas-liquid interface 32, so that the droplets 4 in the reaction chamber 11 flow out of the reaction chamber 11 to the droplet generating structure, and external pressure drives the detection separation oil to enter the droplet generating structure from the oil-liquid interface 31. The detection separation oil separates the droplets 4 flowing out of the reaction chamber 11 to the droplet generating structure into a queue, which enters the fluorescence detection area 33. In this technical solution, the chip body 1 is integrated with a sample adding chamber 12, a reaction chamber 11, a droplet generating structure and a fluorescence detection area 33, so that the generation and detection of the droplets are integrated on the same chip, and the degree of integration and automation can be improved; more importantly, through time-sharing multiplexing of the droplet generating structure (with the flipping of the integrated droplet chip as the time-sharing boundary), droplet generation, amplification and detection are integrated into one chip, realizing a fully integrated and fully closed digital PCR process, which not only inherits the advantages of uniform droplet size, unrestricted droplet number, and high fluorescence detection signal-to-noise ratio, but also overcomes the difficulties of the original chip structure being complex, generation and detection being completed in different chips, low integration, and difficulty in automation. It is an important technical breakthrough in the field of digital PCR.

As a specific embodiment of the droplet generating structure, the droplet generating structure includes an oil-liquid pipeline 21 and a connecting pipeline, the oil-liquid pipeline 21 and the connecting pipeline form a cross, the connecting pipeline includes a first pipeline 22 located on the first side of the cross intersection and connected to the reaction chamber 11 and a second pipeline 23 located on the second side of the cross intersection and connected to the sample adding chamber 12, and the oil-liquid interface 31 is connected to the oil-liquid pipeline 21.

In some embodiments of the integrated droplet chip, as shown in FIG1 , with the first side surface of the chip body 1 being in a horizontal position as a reference, the reaction chamber 11 and the sample adding chamber 12 are on the first side surface, and the connecting interface 111 between the reaction chamber 11 and the first side surface of the chip body 1 extends upwards and forms a flared mouth with a small upper part and a large lower part. The flared mouth connecting interface 111 can facilitate the droplets 4 to enter the reaction chamber 11 from the first pipeline 22, and also facilitate the droplets 4 to enter the first pipeline 22 from the reaction chamber 11, thereby preventing the droplets 4 from being detained. It should be noted that at this time, the reaction chamber 11 and the sample adding chamber 12 are both located on the first side surface (specifically the top surface) of the chip body 1, and the droplets 4 entering the reaction chamber 11 are all gathered at the connecting interface 111. When performing PCR amplification, the chip body 1 needs to be inverted as a whole, that is, flipped 180°, so that the droplets 4 can be in the reaction area of the reaction chamber 11.

In some embodiments, a gas-liquid pipeline 112 extending from bottom to top is further constructed in the reaction chamber 11, and the lower end of the gas-liquid pipeline 112 is connected to the gas-liquid interface 32, and the upper end of the gas-liquid pipeline 112 is higher than the upper end of the connecting interface 111. This can prevent the droplets 4 generated by the droplet generating structure from further flowing out of the gas-liquid pipeline 112 after entering the reaction chamber 11 when the reaction chamber 11 is under negative pressure.

In some embodiments, a droplet observation area 34 is provided between the first pipeline 22 and the connecting interface 111, and the flow area of the droplet observation area 34 is much larger than the flow area of the first pipeline 22, that is, the droplet observation area 34 is an area enlarged on the first pipeline 22 (the width becomes larger), so that the flow rate of the droplets 4 entering the area is reduced, which can facilitate external camera imaging, record the droplet morphology, and determine whether the state of the droplet generation process is normal.

As a specific embodiment, the sample adding chamber 12 includes an opening chamber 121 and a sealing cover 122 sealedly connected to the opening of the opening chamber 121, so that an operator can add a sample into the sample adding chamber 12. Furthermore, the sample adding chamber 12 is provided with a filter membrane or an exhaust hole. When the sample adding chamber 12 becomes a waste liquid pool (that is, when the droplet chip is turned upside down), a certain amount of air is exhausted to prevent pressure accumulation in the sample adding chamber 12. In one embodiment, the fluorescence detection area 33 is located on the second pipeline 23. On the second pipeline 23, the droplets 4 flowing out of the reaction chamber 11 can be separated into droplet queues with appropriate spacing under the action of the detection pushing oil in the oil-liquid pipeline 21 when passing through the cross point, thereby completing the fluorescence detection under the action of the external system.

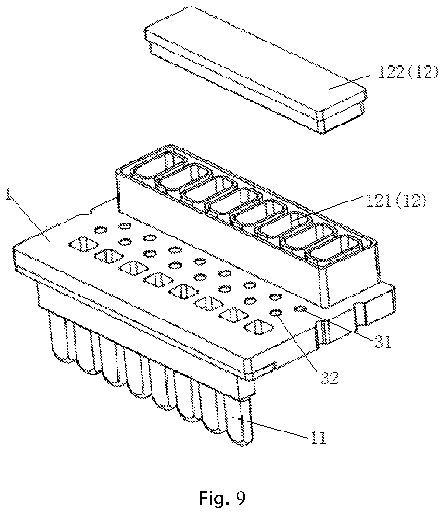

As shown in FIG 9 , another embodiment of the integrated droplet chip is given, which is different from the integrated droplet chip shown in FIG1 in that the reaction chamber 11 and the sample adding chamber 12 are respectively located on two opposite sides of the chip body 1. Specifically, the sample adding chamber 12 is located on the first side, and the reaction chamber 11 is located on the second side. The second side and the first side are opposite sides of the chip body 1. At this time, the working principle and process of the integrated droplet chip are basically the same as those of the integrated droplet chip described above. The difference is that during the droplet generation process, since the reaction chamber 11 is located on the bottom side of the chip body 1 (the sample adding chamber 12 is on the top side), the droplet 4 will directly fall into the reaction zone at the bottom of the reaction chamber 11 when it enters the reaction chamber 11, and be collected in the reaction area. Therefore, after the droplet generation is completed, the integrated droplet chip does not need to be flipped 180° and can directly enter the subsequent amplification process.

In some embodiments, before the droplets 4 enter the reaction chamber 11, light oil (i.e., oil with a lower density) is preset in the reaction chamber 11 to ensure that the light oil can always be on the top of the droplets 4 in the reaction chamber 11, thereby solving the problem of droplet evaporation during amplification and realizing heat-cover-free PCR.

According to an embodiment of the present invention, a digital PCR method for an integrated droplet chip is also provided. The integrated droplet chip is as described above, comprising a chip body 1, wherein the chip body 1 has a reaction chamber 11 and a sample adding chamber 12, wherein a droplet generating structure, an oil-liquid interface 31, a gas-liquid interface 32 and a fluorescence detection area 33 are constructed in the chip body 1, wherein the gas-liquid interface 32 is connected to the reaction chamber 11, the sample adding chamber 12 is connected to the droplet generating structure, and the oil-liquid interface 31 is connected to the droplet generating structure.

The digital PCR method comprises the following steps:
The step of segmenting and generating droplets is to control the formation of a first pressure difference between the sample adding chamber 12 and the gas-liquid interface 32, and a second pressure difference between the oil-liquid interface 31 and the gas-liquid interface 32, so that the first pressure difference and the second pressure difference respectively drive the sample in the sample adding chamber 12 and the generated oil of the oil-liquid interface 31 to enter the droplet generating structure, and the generated droplets 4 enter and are stored in the reaction chamber 11. Specifically, oil is provided to the oil-liquid interface 31, and negative pressure is provided to the gas-liquid interface 32. Under the action of the negative pressure, the sample in the sample adding chamber 12 and the oil at the oil-liquid interface 31 are respectively driven to converge along the second pipeline 23 and the oil-liquid pipeline 21 to the cross intersection of the droplet generating structure. Under the action of the shear force and surface tension of the oil fluid, the sample forms droplets 4 (water-in-oil droplets) of uniform size, and finally, under the action of the negative pressure, the droplets 4 enter the reaction chamber 11 through the first pipeline 22 for storage. It should be noted that when the droplets enter the droplet observation area 34, the flow rate of the droplets 4 forms a dense droplet community, which is convenient for camera imaging and recording.

In the amplification reaction step, the reaction chamber 11 is placed in a heating module (not shown) and amplified by heating according to a preset cycle. The heating module can be an existing heating module.

The droplet detection step is to control the external pressure to drive the detection pushing oil 5 to enter the reaction chamber 11 from the gas-liquid interface 32, so that the droplets 4 in the reaction chamber 11 flow out of the reaction chamber 11 to the droplet generating structure, and the external pressure drives the detection separation oil to enter the droplet generating structure from the oil-liquid interface 31. The detection separation oil separates the droplets 4 flowing out of the reaction chamber 11 to the droplet generating structure into a queue, and enters the fluorescence detection area 33 to complete the fluorescence detection. Specifically, the gas-liquid interface 32 oil (i.e., detection driving oil 5, also called floating oil) is provided, and the droplets 4 after the amplification reaction in the reaction chamber 11 are floated by the buoyancy of the oil. Under the buoyancy of the oil, the droplets 4 can flow out of the reaction chamber 11 through the connecting interface 111 and enter the first pipeline 22, and flow through the droplet observation area 34 to enter the cross intersection, and enter the second pipeline 23, and are detected in the fluorescence detection area 33, and then finally enter the sample adding chamber 12. At this time, the sample adding chamber 12 is a waste liquid pool.

In this technical solution, droplet generation, amplification and detection are integrated into one chip through time-sharing multiplexing of the droplet generating structure (with the flipping of the integrated droplet chip as the time-sharing boundary), thereby realizing a fully integrated and fully closed digital PCR process. This not only inherits the advantages of uniform droplet size, unrestricted droplet number, and high fluorescence detection signal-to-noise ratio, but also overcomes the difficulties of the original chip structure being complex, generation and detection being completed in different chips, low integration, and difficulty in automation. This is an important technological breakthrough in the field of digital PCR.

In some embodiments, with the first side of the chip body 1 being in a horizontal position as a reference, the reaction chamber 11 and the sample adding chamber 12 are on the first side, and before the amplification reaction step and after the droplet segmentation generation step, it also includes: a chip flipping step, controlling the chip body 1 to flip up and down 180°, at this time, the droplets 4 in the reaction chamber 11 are flipped from the side close to the connecting interface 111 to the side away from the connecting interface 111, and the position of the reaction chamber 11 corresponding to the droplet 4 is the reaction area of the reaction chamber 11, and the reaction area is in contact with the heating module for temperature adjustment to realize the temperature regulation reaction of the sample.

The following is a further description of the operation process of the integrated droplet chip of the present invention in conjunction with FIGS. 1 to 8 :
First, 30 µl of the system (ie, the aforementioned sample) is added to the sample adding chamber 12. The 30 µl PCR system includes 10 µl of Bio-Rad's ddPCR Supermix for Probes, 5 µl of GJB2 gene upstream and downstream primer reagents, and 5 µl of a template containing 1 ng of genomic DNA. The whole chip is shown in FIG1 . A chip includes 8 parallel independent droplet chip structures, each of which includes a sample adding chamber 12, an oil-liquid interface 31, a gas-liquid interface 32 , a droplet generating structure, and a reaction chamber 11 .

Then, the sealing cover 122 is tightly closed or bonded to seal the sample adding chamber 12. The sample adding chamber 12 is preferably provided with a filter membrane or a small-caliber exhaust hole.

Then, the Bio-Rad Generation Oil required for droplet generation is provided to the oil-liquid interface 31, wherein the generation oil contains a surfactant that can stabilize the droplets; a negative pressure of -200 mBar is provided to the gas-liquid interface 32 , so that a pressure difference is formed between the sample adding chamber 12 and the oil-liquid interface 31 .

Among them, the sample adding chamber 12 is connected to the second pipeline 23 in the droplet generating structure, the oil-liquid interface 31 is connected to the oil-liquid pipe 21 in the droplet generating structure, the first pipeline 22 and the gas-liquid interface 32 are both connected to the reaction chamber 11, wherein the first pipeline 22 is connected to the connecting interface 111, and the gas-liquid interface 32 is connected to the gas-liquid pipeline 112. The various parts of the droplet generating structure are shown in FIG. 2 . The oil-liquid pipeline 21 has two branches, which are respectively located on both sides of the second pipeline 23 and the first pipeline 22, and are both connected to the oil-liquid interface 31. The first pipeline 22 may include a droplet observation area 34. The pipe in the observation area becomes wider, and the flow rate of the droplets decreases after entering, which can facilitate external camera imaging, record the droplet morphology, and determine whether the state of the droplet generation process is normal.

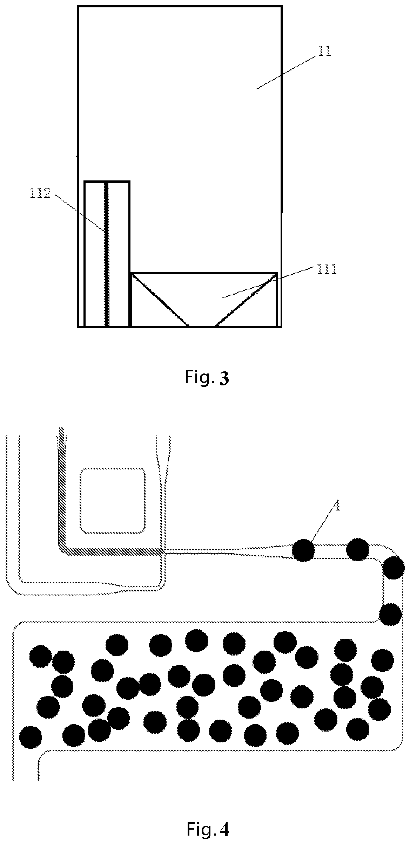

Driven by the pressure difference, the reaction system enters the second pipeline 23, and the generated oil enters the oil-liquid pipeline 21 and intersects at the cross structure (i.e. the aforementioned cross intersection), and under the action of fluid shear force and surface tension, oil-in-water droplets 4 of uniform size are formed. The channel depth at the cross is about 70 µm, the width is 80 µm, and the droplet size is about 100 µm. The droplets 4 enter the first pipeline 22, and reduce the flow rate after entering the droplet observation area 34, forming a dense droplet community, which is convenient for camera imaging and recording. The principle diagram of the droplet generation process is shown in Figure 4.

The generated droplets flow through the first pipeline 22 and then reach the connecting interface 111 of the reaction chamber 11. The bottom of the connecting interface 111 has a slope structure (ie, the aforementioned flared mouth), and the bottom of the slope is connected to the first pipeline 22. When the droplet generation process is finished, the pressure difference applied to the chip interface is removed, and the droplet should still be located below the gas-liquid pipeline 112.

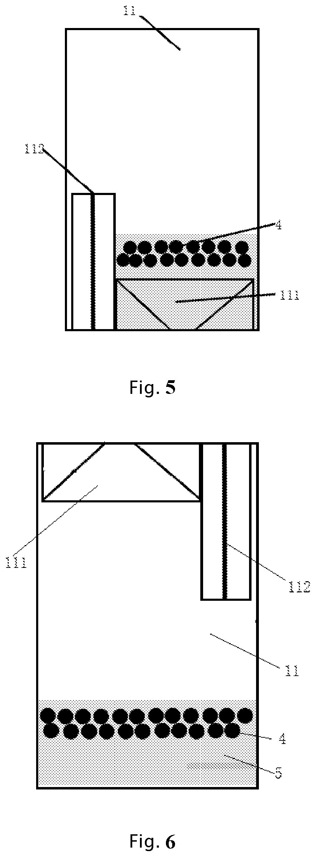

Afterwards, the chip is turned upside down to transfer the droplets from the connecting interface 111 to the reaction area (ie, away from the connecting interface 111 ), as shown in FIG. 5 . The structure of the reaction area should be designed with high heat transfer efficiency, such as a flat design with high depth and thin thickness, so that the external system (i.e., the heating module) can heat and cool the reaction zone from the left and right sides, which not only ensures a short temperature conduction distance, but also a large contact area, thereby achieving efficient heat transfer. In this embodiment, the temperature cycling process is to first perform a pre-denaturation at 95°C for 10 minutes, followed by 40 temperature cycles, with each cycle being 95°C for 5 seconds, 60°C for 15 seconds, and finally keeping warm at 4°C. In order to reduce evaporation, 30 µl of a low-density anti-evaporation reagent may be placed in the reaction chamber 11 in advance.

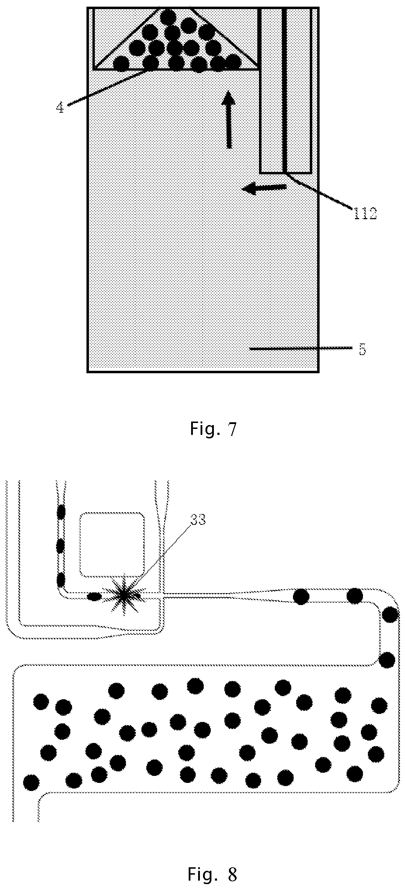

After the temperature cycle is completed, the amplification reaction in the droplet containing the template is also completed accordingly, and it is necessary to enter the droplet fluorescence detection stage. The detection pushing oil (ie, the detection driving oil 5 mentioned above) is injected into the gas-liquid interface 32 to continuously fill the reaction chamber 11. In this process, the liquid level of the droplets continues to rise, and then is guided by the slope of the connecting interface 111 and enters the first pipeline 22 . This process is shown in Figure 7.

When the droplets pass through the observation area in the first pipeline 22, the camera can also be used to perform bright field imaging of the droplets, so as to evaluate the state of the droplets after the amplification reaction. At the same time, the detection pushing oil is injected into the oil-liquid interface 31, and the detection pushing oil passes through the oil-liquid pipeline 21, merges with the droplet queue at the cross pipeline, and separates the closely arranged droplets into droplet queues with appropriate spacing. The droplet queue passes through the fluorescence detection area 33 located in the second pipeline 23 in sequence, as shown in FIG. 8. The position corresponding to the fluorescence detection area 33 is the fluorescence detection focus of the external system. An external system focuses excitation light, such as laser or LED narrowband light at wavelengths of 488nm and 532nm, to the detection focus. As the droplets pass through the detection focus one by one, the fluorescence excited in the droplets will also be received by the collection light path of the external system, thereby obtaining the fluorescence information of each droplet. The fluorescence information of the droplets is used to define the signal threshold, distinguish the positive and negative nature of the droplets, and the Poisson distribution model is used to calculate the copy number of the target molecule in the sample.

Finally, the droplets that have completed the fluorescence detection enter the sample adding chamber 12. Since the sample adding chamber 12 has been sealed by the sealing cover 122, it will not come into contact with the environment outside the chip, eliminating the possibility of aerosol contamination and realizing a fully closed digital PCR process.

In this embodiment, a method of time-division multiplexing of the droplet generating structure is innovatively adopted. When droplets are generated, a droplet generating structure is used to achieve droplet generation. During droplet fluorescence detection, the droplet generating structure completes the separation of the droplet queues, ensuring the detection of droplet fluorescence signals. By using this time-sharing multiplexing method, it was realized for the first time in the flow-like digital PCR technology route that a single chip can complete the fully integrated and closed digital PCR process of droplet generation, amplification, and detection, which is an important technological breakthrough in the field of digital PCR.

In another embodiment, when the droplets pass through the observation area in the first pipeline 22, the droplets are fluorescently imaged by a camera, and the imaged photos are analyzed to obtain the fluorescence intensity information of each droplet in the photo, thereby completing the droplet fluorescence signal detection.

It is easy for those skilled in the art to understand that, under the premise of no conflict, the above-mentioned advantageous methods can be freely combined and superimposed.

The above are only preferred embodiments of the present invention and are not intended to limit the present invention. Any modifications, equivalent substitutions and improvements made within the spirit and principles of the present invention should be included in the protection scope of the present invention. The above are only preferred embodiments of the present invention. It should be pointed out that, for ordinary technicians in this technical field, several improvements and modifications can be made without departing from the technical principles of the present invention. These improvements and modifications should also be regarded as the protection scope of the present invention.

## Claims

1. An integrated droplet chip, **characterized in that** it comprises a chip body (1), the chip body (1) has a reaction chamber (11) and a sample adding chamber (12), and said chip body (1) being constructed with droplet generating structure, oil-liquid interface (31), gas-liquid interface (32) and fluorescence detection area (33), the gas-liquid interface (32) communicates with the reaction chamber (11), and the sample adding chamber (12) communicates with the droplet generating structure, and the oil-liquid interface (31) communicates with the droplet generating structure;
when the droplets are generated, a first pressure difference is formed between the sample adding chamber (12) and the gas-liquid interface (32), and a second pressure difference is formed between the oil-liquid interface (31) and the gas-liquid interface (32), the first pressure difference and the second pressure difference respectively drive the sample in the sample adding chamber (12) and the generated oil of the oil-liquid interface (31) to enter the droplet generating structure, the generated droplets (4) enter and are stored in the reaction chamber (11);
during the droplet detection, the external pressure drives the detection pushing oil (5) to enter the reaction chamber (11) from the gas-liquid interface (32), so that the droplets (4) in the reaction chamber (11) flow out of the reaction chamber (11) and enter into the droplet generating structure, and the external pressure drives the detection separation oil to enter the droplet generating structure from the oil-liquid interface (31), and the detection separation oil separates the droplets (4) flowing out of the reaction chamber (11) into the droplet generating structure to form a queue, which enters the fluorescence detection area (33).

2. The integrated droplet chip according to claim 1, wherein the droplet generating structure comprises an oil-liquid pipeline (21) and a communication pipeline, the oil-liquid pipeline (21) intersects with the communication pipeline in a cross manner, the communication pipeline comprises a first pipeline (22) located on a first side of the cross point and communicated with the reaction chamber (11), and a second pipeline (23) located on a second side of the cross point and communicated with the sample adding chamber (12), and the oil-liquid interface (31) is communicated with the oil-liquid pipeline (21).

3. The integrated droplet chip according to claim 2, wherein the reaction chamber (11) and the sample adding chamber (12) are located on the first side surface with reference to the first side surface of the chip body (1) in a horizontal direction, and the connecting interface (111) between the reaction chamber (11) and the first side of the chip body (1) extending upwards and forming a flared mouth with a small upper part and a large lower part.

4. The integrated droplet chip according to claim 3, wherein a gas-liquid pipeline (112) extending from bottom to top is further configured in the reaction chamber (11), a lower opening of the gas-liquid pipeline (112) is in communication with the gas-liquid interface (32), and an upper opening of the gas-liquid pipeline (112) is higher than an upper opening of the connecting interface (111).

5. The integrated droplet chip according to claim 4, wherein a droplet observation area (34) is provided between the first pipeline (22) and the connecting interface (111).

6. The integrated droplet chip according to claim 1, wherein the sample adding chamber (12) comprises an opening chamber (121) and a sealing cover (122) hermetically connected to said opening chamber (121).

7. The integrated droplet chip according to claim 1, wherein the sample adding chamber (12) is provided with a filter membrane or an exhaust hole.

8. The integrated droplet chip according to claim 2, wherein the fluorescence detection area (33) is located on the second pipeline (23).

9. The integrated droplet chip according to claim 2, wherein the sample adding chamber (12) is on a first side surface and the reaction chamber (11) is on a second side surface, said first side surface and said second side surface being opposite sides of said chip body (1).

10. The integrated droplet chip according to claim 1, wherein light oil is preset in the reaction chamber (11) before the droplets (4) enter the reaction chamber (11).
